# EUROPÄISCHE PATENTANMELDUNG

(11) **EP 4 661 015 A1**
(43) Veröffentlichungstag der Anmeldung: **10.12.2025**
(21) Anmeldenummer: 24180645.4
(22) Anmeldetag: 07.06.2024
(51) Int. Cl.: G16H 10/40, G06K 7/14, G06K 17/00, G16H 10/65, G16H 20/17, A61J 1/05

(54) **VERFAHREN ZUR PRÜFUNG EINER ZUORDNUNG EINES BLUTPRODUKTS ZU EINEM PATIENTEN, ENDGERÄT UND COMPUTERPROGRAMM**

(71) Anmelder: Friedrich-Alexander-Universität Erlangen-Nürnberg, 91054 Erlangen (DE)
(72) Erfinder: HACKSTEIN, Holger, 35398 Gießen (DE)
(74) Vertreter: FDST Patentanwälte

(57) **Zusammenfassung**

Es wird ein Verfahren zur Prüfung einer Zuordnung eines Blutprodukts (2) zu einem Patienten (4) angegeben, wobei das Blutprodukt (2) mit einem Kennzeichnungselement (14) gekennzeichnet ist, welches Produktdaten (16) des Blutprodukts (2) enthält, wobei ein Endgerät (10) sowohl die Produktdaten (16) erfasst als auch Patientendaten (20), prüft, ob die Produktdaten (16) und die Patientendaten (20) zueinander passen, eine positive Meldung (24) ausgibt, wenn die Produktdaten (16) zu den Patientendaten (20) passen, und ansonsten eine negative Meldung (26). Weiter werden ein Endgerät (10) und ein Computerprogramm angegeben.

## Beschreibung

Die Erfindung betrifft ein Verfahren zur Prüfung einer Zuordnung eines Blutprodukts zu einem Patienten. Weiter betrifft die Erfindung ein Endgerät und ein Computerprogramm in diesem Zusammenhang.

Bei einer Transfusion (auch Bluttransfusion) wird einem Patienten ein Blutprodukt verabreicht. Eine solche Transfusion wird regelmäßig von einem Arzt durchgeführt. Der Arzt ist typischerweise auch angehalten, zu prüfen, ob das konkret vorliegende Blutprodukt auch zu dem konkret vorliegenden Patienten passt; mit anderen Worten: ob für die bevorstehende Transfusion das korrekte Blutprodukt bereitgestellt wurde. Dies ist erforderlich, da die Verabreichung eines falschen Blutprodukts für den Patienten tödlich sein kann und sich sowohl bei der Bereitstellung des Blutprodukts beim Patienten als auch schon zuvor bei der Zuordnung des Blutprodukts zum Patienten verschiedene Fehler ergeben können. Beispielsweise wird aus einem lokalen Lagerschrank auf einer Station versehentlich ein falsches Blutprodukt gegriffen und beim Patienten bereitgelegt und dieser Fehler vom Arzt übersehen, da dieser auf die korrekte Bereitstellung vertraut. Das Ergebnis ist eine potentiell tödliche Fehltransfusion. Möglich ist auch eine Verwechslung von Begleitdokumenten zu verschiedenen Blutprodukten.

Es wird verwiesen auf die "Richtlinie zur Gewinnung von Blut und Blutbestandteilen und zur Anwendung von Blutprodukten (Hämotherapie-Richtlinie) Bundesärztekammer (BÄK), Deutscher Ärzteverlag 2023 ISBN 978-3-7691-3807-8 und auf Knels R, Mönig HJ, Wittmann G, von Versen R, Pruss A. "Eurocode International Blood Labeling System" enables unique identification of all biological products from human origin in accordance with the European Directive 2004/23/EC. Cell Tissue Bank. 2010 Nov; 11 (4 ):345-52.

Weiterhin wird verwiesen auf die in https://hospital.haemonetics.com/-/media/files/hospital/col-copy-001182-us bloodtrack brochure.pdf beschriebene Lösung zu Blutmanagement und Transfusion am Patientenbett ("Blood Management and Bedside Transfusion Solution").

Vor diesem Hintergrund ist es eine Aufgabe der Erfindung, das Risiko einer Fehltransfusion soweit wie möglich zu reduzieren. Insbesondere soll ein Arzt, welcher eine Transfusion durchführt, bei der Entscheidungsfindung unterstützt werden, ob in einem jeweils konkreten Fall das vorliegende Blutprodukt dem Patienten verabreicht werden kann. Hierzu sollen ein entsprechendes Verfahren, ein Endgerät und ein Computerprogramm angegeben werden.

Die Aufgabe wird erfindungsgemäß gelöst durch ein Verfahren mit den Merkmalen gemäß Anspruch 1, durch ein Endgerät mit den Merkmalen gemäß Anspruch 11 und ein Computerprogramm mit den Merkmalen gemäß Anspruch 13. Vorteilhafte Ausgestaltungen, Weiterbildungen und Varianten sind Gegenstand der Unteransprüche. Die Ausführungen im Zusammenhang mit dem Verfahren gelten sinngemäß auch für das Endgerät und das Computerprogramm sowie umgekehrt. Sofern nachfolgend Schritte des Verfahrens beschrieben sind, ergeben sich vorteilhafte Ausgestaltungen für das Endgerät dadurch, dass dieses ausgebildet ist, einen oder mehrere dieser Schritte auszuführen, und vorteilhafte Ausgestaltungen für das Computerprogramm ergeben sich dadurch, dass dieses Befehle aufweist, welche bewirken, dass ein Endgerät (auf welchem das Computerprogramm ausgeführt wird) einen oder mehrere dieser Schritte ausführt.

Ein Kernaspekt der Erfindung ist insbesondere ein elektronisch assistiertes, vorzugsweise visuelles, Prüfverfahren, um Verwechslungen von Transfusionen direkt am Patientenbett zu verhindern (Verwechslungsproblem). Dadurch werden Fehltransfusionen verhindert. Die Erfindung ist konkret nutzbar im Zusammenhang mit Prozessen und Abläufen in einem Krankenhaus und allgemein überall dort, wo im Vorfeld einer Transfusion Daten abgeglichen werden sollen.

Das erfindungsgemäße Verfahren dient zur Prüfung einer Zuordnung eines Blutprodukts zu einem Patienten. Dies wird auch als Integritätsprüfung oder Validierung bezeichnet. Das Blutprodukt ist mit einem Kennzeichnungselement gekennzeichnet, welches Produktdaten des Blutprodukts enthält. Genauer gesagt ist das Blutprodukt in einem Behältnis, z.B. Beutel, aufbewahrt (abgepacktes Blutprodukt) und das Kennzeichnungselement ist an diesem Behältnis angebracht oder darin integriert. Das Kennzeichnungselement ist dem Blutprodukt insbesondere fest zugeordnet, d.h. fest oder nicht lösbar an dem Behältnis angebracht, z.B. aufgeklebt, oder darin integriert. Das Kennzeichnungselement ist allgemein ein Datenträger, geeigneterweise ein Etikett, welches auf das Behältnis aufgeklebt ist. Die Produktdaten und insgesamt jegliche Daten, welche in dem Kennzeichenelement enthalten sind, sind insbesondere nicht-personalisiert, enthalten also keinerlei Patientendaten.

Ein Endgerät erfasst nun in einem ersten Verfahrensschritt sowohl die Produktdaten als auch Patientendaten. In einem zweiten Verfahrensschritt prüft das Endgerät, ob die Produktdaten und die Patientendaten zueinander passen. Das Endgerät führt demnach eine Prüfung der erfassten Daten durch. In einem dritten Verfahrensschritt gibt das Endgerät eine positive Meldung (Prüfung bestanden, Zuordnung korrekt) aus, wenn die Produktdaten zu den Patientendaten passen, und ansonsten eine negative Meldung (Prüfung nicht bestanden, Zuordnung nicht korrekt). Das eigentliche Verfahren wird somit von dem Endgerät ausgeführt.

Als Endgerät eignet sich insbesondere jeder Computer, welcher eingerichtet ist, die beschriebene Erfassung, Prüfung und Ausgabe durchzuführen. Entsprechend ist das Verfahren insbesondere ein computerimplementiertes Verfahren. Die hier vorgestellte Erfindung ist auch nicht einfach nur ein automatisiertes Abgleichen von Daten, vielmehr ist ein wichtiger Aspekt, dass sowohl das Erfassen und Prüfen der Daten als auch das Ausgeben der Meldung mittels eines einzelnen Endgeräts erfolgt, welches selbstständig arbeitet und unabhängig ist von übrigen Geräten, speziell Krankenhausinfrastruktur. Das Endgerät muss insbesondere lediglich von einem Bediener, z.B. einem Arzt, jeweils so ausgerichtet und/oder bedient werden, dass dieses die Daten erfassen kann. Das Endgerät ist also eine vorteilhafte "stand-alone"-Lösung. Entsprechend benötigt das Endgerät in einer vorteilhaften Ausgestaltung für die Erfassung und für die Prüfung keine externe Datenbank und greift auf eine solche auch nicht zu. Alternativ oder zusätzlich benötigt das Endgerät vorteilhafterweise für die Erfassung und für die Prüfung analog kein externes Gerät und greift auf ein solches auch nicht zu. Das Verfahren und das Endgerät sind auch insgesamt insbesondere dazu vorgesehen und ausgebildet, in räumlicher Nähe des Patienten eingesetzt zu werden (eine diagnostische oder therapeutische Interaktion mit dem Körper des Patienten erfolgt aber nicht). Vorzugsweise wird das Verfahren als Abschluss einer Vorbereitung der Transfusion und/oder zeitlich unmittelbar vor dieser Transfusion durchgeführt.

Durch das Endgerät wird ein Arzt vorteilhaft bei der Entscheidungsfindung unterstützt, ob ein am Patientenbett bereitgestelltes Blutprodukt transfundiert werden kann oder nicht. Das Endgerät wird dabei insbesondere vom Arzt bedient. Die Unterstützung besteht insbesondere darin, dass das Endgerät die verschiedenen zu prüfenden Daten, nämlich wenigstens die Produktdaten des Blutprodukts und Patientendaten zum Patienten, erfasst und miteinander abgleicht und dann eine entsprechende Meldung ausgibt. Die Meldung wird insbesondere auch als "Transfusionsempfehlung" bezeichnet. Im Kern führt das Endgerät somit automatisiert eine Integritätsprüfung der vor Ort vorliegenden Daten durch, d.h. das Endgerät prüft, ob die Produktdaten und die Patientendaten zueinander passen. Damit wird anhand der Daten effektiv geprüft, ob dem Patienten letztendlich das korrekte Blutprodukt zur Transfusion zugeordnet wurde. Abhängig vom Ergebnis der Prüfung gibt das Endgerät eine entsprechende Meldung aus, welche der Arzt dann bei der Entscheidung, ob das Blutprodukt transfundiert wird oder nicht, verwenden kann. Die tatsächliche Entscheidung, ob eine Transfusion erfolgt, und auch die tatsächliche Transfusion, werden jedoch vom Arzt vorgenommen, das Endgerät ist hieran nicht beteiligt, d.h. die tatsächliche Entscheidung und die Durchführung der Transfusion sind nicht Teil des hier beschriebenen Verfahrens. Das Verfahren umfasst auch allgemein keinerlei Diagnose oder Therapie am menschlichen oder tierischen Körper.

In einer besonders bevorzugten Ausgestaltung weist das Endgerät eine optische Leseeinheit, insbesondere eine Kamera, auf, mit welchem die Produktdaten und/oder die Patientendaten erfasst werden. Hierbei wird insbesondere der jeweilige Datenträger (z.B. Etikett, Patientenarmband, Begleitdokument) mit der Leseeinheit gescannt oder fotografiert. Vorzugsweise werden sämtliche Daten, welche bei der nachfolgenden Prüfung verwendet werden, mittels desselben Endgeräts, gegebenenfalls sogar mittels derselben Leseeinheit erfasst. Die Produktdaten und die Patientendaten liegen ganz oder teilweise insbesondere in Form von einem oder mehreren maschinenlesbaren Codes (nicht unmittelbar menschenlesbar), insbesondere Barcodes oder Vergleichbare, oder von menschenlesbarem Text (Zeichenketten oder Ähnliches, mittelbar maschinenlesbar nur mit zusätzlicher Texterkennung) vor oder eine Kombination hiervon. Insbesondere sind die Produktdaten und die Patientendaten gedruckte Daten, z.B. auf ein Papierblatt, ein Etikett, ein Patientenarmband aufgedruckt. Insgesamt sind die Daten somit generell visuell verfügbar und können daher auf besonders einfache Weise mittels einer einfachen optischen Leseeinheit erfasst werden. Eine Kamera eignet sich hierbei ganz besonders als Leseeinheit, da diese die Erfassung von Daten in unterschiedlicher Darstellung ermöglicht, nämlich sowohl in maschinenlesbarer Form als auch in menschenlesbarer Form. Für ersteren Fall weist das Endgerät insbesondere eine Codeerkennung auf, welche die in maschinenlesbarer Form vorliegenden Daten in einem mit der Kamera aufgenommenen Bild erkennt und für die Prüfung erfasst. Für letzteren Fall weist das Endgerät insbesondere eine Texterkennung auf, welche die in menschenlesbarer Form vorliegenden Daten in einem mit der Kamera aufgenommenen Bild erkennt und für die Prüfung in ein vom Endgerät verwertbares Format übersetzt. Anstelle einer optischen Leseeinheit sind grundsätzlich auch andere Leseeinheit geeignet, z.B. RFID- oder NFC- Leseeinheit, welche die Daten aus einem entsprechenden Tag auslesen und in das Endgerät integriert sind.

Grundsätzlich ist es ausreichend, wenn die Produktdaten und/oder die Patientendaten entweder in Form eines (d.h. wenigstens eines) maschinenlesbaren Codes vorliegen, welcher vom Endgerät eingelesen wird, oder in Form von menschenlesbarem Text. In einer vorteilhaften Ausgestaltung ist jedoch beides der Fall, d.h. die Produktdaten und/oder die Patientendaten liegen in Form eines maschinenlesbaren Codes und zugleich auch in Form von menschenlesbarem Text vor, sodass die Produktdaten und/oder die Patientendaten vom Endgerät redundant auf wenigstens zwei verschiedene Weisen erfasst werden. Entsprechend ist dann vorteilhafterweise auch die Prüfung, welche das Endgerät durchführt, redundant. Dabei ist eine redundante Prüfung vorteilhaft auf zweierlei Weise möglich. In einer Ausgestaltung prüft das Endgerät einen redundant in derselben Datenquelle (Kennzeichnungselement, Identifikationselement, Begleitdokument oder manuelle Eingabe) enthaltenen Parameter und somit die Integrität der Daten dieser Datenquelle. Beispielsweise wird die Produkt-ID in dem Begleitdokument in Form eines maschinenlesbaren Codes verglichen mit der Produkt-ID in dem Begleitdokument in Form von menschenlesbarem Text (Code-zu-Text-Vergleich innerhalb derselben Datenquelle). Alternativ oder zusätzlich werden jeweils gleichförmig vorliegende Parameter zweier unterschiedlicher Datenquellen verglichen, d.h. das Endgerät prüft einen Parameter in Form eines maschinenlesbaren Codes in zwei unterschiedlichen Datenquellen und zusätzlich den gleichen Parameter in Form von menschenlesbarem Text in den zwei Datenquellen. Beispielsweise enthalten sowohl das Begleitdokument als auch das Kennzeichnungselement die Produkt-Blutgruppe jeweils sowohl in Form eines maschinenlesbaren Codes als auch in Form von menschenlesbarem Text. Das Endgerät prüft dann, ob die Produkt-Blutgruppe als Code in dem Begleitdokument dem Code für die Produkt-Blutgruppe im Kennzeichnungselement entspricht und zusätzlich auch, ob die Produkt-Blutgruppe als Text in dem Begleitdokument dem Text für die Produkt-Blutgruppe im Kennzeichnungselement entspricht.

Alternativ oder zusätzlich weist das Endgerät ein Eingabeelement auf, z.B. Touchscreen, über welches zumindest ein Teil der Daten manuell eingegeben werden kann und dann als manuelle Eingabe erfasst wird. Dies ist besonders vorteilhaft zur Erfassung des Ergebnisses eines AB0-Test zur Bestimmung der Patienten-Blutgruppe am Patientenbett (AB0-Bedside-Test), da eine Auswertung mittels Bilderkennung bei einer Erfassung mittels Kamera zwar grundsätzlich möglich, aber typischerweise schwierig ist. Grundsätzlich können auch alle anderen Daten als manuelle Eingabe erfasst werden, bevorzugt ist aber eine Erfassung durch das Endgerät selbst wie oben beschrieben, da hierdurch Eingabefehler ausgeschlossen werden.

Besonders bevorzugt ist auch eine Ausgestaltung, bei welcher das Endgerät ein mobiles Endgerät (Mobilgerät) ist, insbesondere Smartphone, Tablet, PDA (personal digital assistant) oder dergleichen. Ein mobiles Endgerät ist ein Endgerät, welches aufgrund von dessen Größe und Gewicht ohne größere körperliche Anstrengung tragbar und somit mobil einsetzbar ist. Ein mobiles Endgerät ist insbesondere auch im Betrieb selbst noch mobil. Besonders bevorzugt ist ein Smartphone, da ein solches ohnehin von den meisten Personen mitgeführt wird und gut und einfach verfügbar ist. Das ermöglicht vorteilhaft sogar, dass medizinisches Personal ein privates mobiles Endgerät nutzt und das Krankenhaus selbst keine Hardware kosten tragen muss. Das mobile Endgerät gehört demnach nicht zwingend zur Krankenhausinfrastruktur, sondern ist in einer möglichen Ausgestaltung ein persönliches mobiles Endgerät, welches einer einzelnen Person zugeordnet ist und gehört, z.B. einem Arzt. Ein mobiles Endgerät weist zudem typischerweise eine für die Erfassung der Daten geeignete Kamera auf und wird mittels eines entsprechenden Computerprogramms (z.B. App) ertüchtigt, die beschriebenen Schritte (Erfassen, Prüfen, Ausgeben) auszuführen. Ein Teil des Computerprogramms ist optional und nach Bedarf auch eine entsprechende Texterkennung oder Codeerkennung (z.B. Barcode-)Lesefähigkeit oder dergleichen für die Erfassung der Daten.

Die erfassten Daten und/oder das Ergebnis der Prüfung werden gegebenenfalls auch gespeichert (Protokollierungsfunktion), insbesondere auf dem Endgerät, dies ist aber nicht zwingend erforderlich. Um speziell im Fall der Nutzung eines privaten mobilen Endgeräts, aber auch ganz allgemein, gegebenenfalls lokal geltende datenschutzrechtliche Anforderungen zu erfüllen, werden die erfassten Daten entsprechend verarbeitet, z.B. werden die Daten vollständig verschlüsselt und mit Zugangskontrolle auf dem Endgerät gespeichert (data containment). Alternativ werden nur die Produktdaten verschlüsselt oder unverschlüsselt isoliert gespeichert und die Personeninformationen, speziell personenbezogene Daten, werden dagegen nicht gespeichert, das Endgerät protokolliert jedoch Zeitangaben wir Datum und Uhrzeit der Erfassung und Prüfung. Alternativ verzichtet das Endgerät vollständig auf die Speicherung der Daten, sodass nur eine Lese- und Prüffunktion (Erfassen und Prüfen) in Verbindung mit der Ausgabe einer Meldung realisiert ist, nicht jedoch eine Protokollierungsfunktion.

Die Erfindung geht aus von der Tatsache, dass Bluttransfusionen ein fester Bestandteil der Arzneimitteltherapie in praktisch jedem Krankenhaus und in vielen Arztpraxen sind. Eine Bluttransfusion ist die Verabreichung eines Blutprodukts an einen Patienten. Der Begriff "Blutprodukt" ist durch das deutsche Transfusionsgesetz sowie das deutsche Arzneimittelgesetz definiert. Beispiele für Blutprodukte sind Erythrozytenkonzentrat, umgangssprachlich als Blutkonserve bezeichnet, Thrombozytenkonzentrat, Frischplasma oder Blutplasmaderivate. Allein in Deutschland werden pro Jahr etwa 4 Millionen Blutprodukte transfundiert. Zur Vermeidung von Verwechslungen bei Bluttransfusionen werden detaillierte Vorschriften mit entsprechenden Kontrollschritten vorgegeben, da eine Verwechslung je nach Blutgruppenkonstellation zu unerwünschten Wirkungen führen kann. So sind die Regeln für die Anwendung von Blutprodukten in Deutschland rechtsverbindlich in der eingangs bereits genannten Hämotherapie-Richtlinie festgelegt. Zur Kennzeichnung findet sich auf jedem Blutprodukt ein Etikett mit einem oder mehreren Barcodes, welche insbesondere gemäß dem Eurocode IBLS (International Blood Labeling System) standardisiert sind und welche eine eindeutige Identifikation inklusive Produktart des Blutproduktes und Produkt-Blutgruppe (Blutgruppe des Blutprodukts) ermöglichen. Zusätzlich zum eigentlichen, maschinenlesbaren Barcode, d.h. einer Balkenfolge, ist der Inhalt eines jeweiligen Barcodes typischerweise auch in menschenlesbarer Textform angegeben, d.h. als Ziffernfolge, auch Barcode-Nummer.

Blutprodukte sind insgesamt sehr sicher, insbesondere aufgrund strenger Infektionstestung. Dennoch kommt es regelmäßig zu unerwünschten Todesfällen nach Bluttransfusionen. Vorliegend wurde beobachtet, dass die Hauptursache für Todesfälle nach Bluttransfusionen weiterhin - trotz zahlreicher Vorschriften - die Verwechslung von Blutprodukten ist. Vor einer Bluttransfusion werden im Krankenhaus verschiedene Untersuchungen wie z.B. Untersuchungen zur Blutgruppe durchgeführt und für den jeweiligen Patienten wird ein individuelles, verträgliches Blutprodukt als zu transfundierendes Blutprodukt identifiziert und für den Patienten ausgewählt. Diese Auswahl umfasst die Erstellung eines Begleitdokuments für das Blutprodukt. Das Begleitdokument enthält Daten, welche ein konkretes Blutprodukt einem konkreten Patienten zuordnen. Dies ist erforderlich, da Blutprodukte als Arzneimittel gelten, deren Kennzeichnung nicht verändert und auch nicht ergänzt werden darf, vor allem nicht um Patientendaten.

Nach der Auswahl eines verträglichen Blutprodukts wird dieses auf eine periphere Station des Krankenhauses transportiert, dort zur Verabreichung bereitgestellt und vor Ort von einem Arzt transfundiert, d.h. dem Patienten verabreicht. Die Auswahl und die anschließende Bereitstellung des Blutprodukts sind Teile einer Zuordnung des Blutprodukts zu dem Patienten, insbesondere diese Zuordnung insgesamt ist Gegenstand der hier vorgestellten Prüfung. Vor der Transfusion führt der Arzt am Patientenbett zweckmäßigerweise verschiedene Kontrollen durch, damit es nicht zu einer potentiell tödlichen Verwechslung von Blutprodukt und Patient kommt. Zweckmäßigerweise erfolgt dabei eine Identitätssicherung des Patienten, ein Vergleich der Barcode-Nummern des bereitgestellten Blutproduktes und des zu transfundierendem Blutprodukts gemäß dem Begleitdokument, ein Vergleich der Produkt-Blutgruppe des bereitgestellten Blutprodukts, der Produkt-Blutgruppe des zu transfundierendem Blutprodukts gemäß dem Begleitdokument und der Patienten-Blutgruppe (Blutgruppe des Patienten). Das vorliegend vorgestellte, erfindungsgemäße Verfahren automatisiert insbesondere einen oder mehrere der vorgenannten Schritte, indem diese von dem Endgerät durchgeführt werden, nämlich im Rahmen der beschriebenen Prüfung, ob die Produktdaten und die Patientendaten zueinander passen. Die Patienten-Blutgruppe wird zweckmäßigerweise vor Ort vom Arzt nochmals mittels eines Bluttests bestimmt, z.B. mittels eines sogenannten AB0-Bedside-Tests. Schließlich beurteilt der Arzt die Kompatibilität der Patienten-Blutgruppe mit der Produkt-Blutgruppe des bereitgestellten Blutprodukts und vergleicht die am Patientenbett bestimmte Patienten-Blutgruppe mit der Patienten-Blutgruppe auf dem Begleitdokument. Anschließend entscheidet der Arzt auf Grundlage der vorstehend beschriebenen Vergleiche, ob das Blutprodukt transfundiert wird und - sofern die Entscheidung positiv ist - transfundiert schließlich auch das Blutprodukt.

Die technische Umsetzung einer verwechslungsfreien Anwendung von Blutprodukten in Krankenhäusern kann grundsätzlich mittels der beiden zwei nachfolgend beschriebenen Ansätze erfolgen:
Bei dem ersten Ansatz ("partiell-elektronisch"), werden zunächst über verschiedenste elektronische Verfahren und Softwarelösungen die Laboruntersuchungen und Blutprodukte den Patienten mit eindeutigen Fallnummern und anderen personenbezogenen Daten (Personalien) zugeordnet, mit entsprechenden Begleitdokumenten versehen und in den peripheren medizinischen Bereich per Botendienst transportiert, wo die Transfusion dann tatsächlich stattfindet. Die tatsächliche Bluttransfusion findet dann am Patientenbett nach manuellen Kontrollen und Datenvergleichen des transfundierenden Arztes statt. In den allermeisten Krankenhäusern sind solche heterogenen Systeme installiert. Diese gewährleisten aber eine lediglich verwechslungsarme Zuordnung von Blutprodukten zu Patienten und auch nur bis zur Krankenstation, d.h. die Bereitstellung des Blutprodukts am Patienten und somit am Ort der Transfusion ist nicht umfasst und weiterhin fehleranfällig. Ein solches System erfordert vom transfundierenden Arzt am Patientenbett zahlreiche manuelle Abgleiche von Patientendaten, Barcodenummern, Blutgruppeninformationen auf Blutprodukt und Begleitdokumenten.

Bei dem zweiten Ansatz ("voll-elektronisch") wird ein technisch aufwändiges, barcodebasiertes, elektronisches Komplettsystem verwendet, welches in die Krankenhaus-EDV integriert wird und komplett alle Blutprodukte und Patientenproben bereits von der Blutgruppenbestimmung bis zur Transfusion und Zuordnung zum Patienten am Patientenbett barcodebasiert überwacht. Nachteile eines solchen vollelektronischen, 360°-barcodebasierten Systems sind hohe Installations- und Betriebskosten, sowie die aufwändige erforderliche komplette Ein- und Anbindung des Systems mit Softwaremodulen und Hardware-Schnittstellen (auch Hardware, inkl. Kühlschränke, Drucker) in allen
Bereichen des Krankenhauses, in welchen Blut transfundiert werden soll.

Trotz zahlreicher Anstrengungen besteht somit das technische Problem der Verwechslung von Blutprodukten am Patientenbett fort und solche Verwechslungen stellen sogar eine Hauptursache für unerwünschte Todesfälle nach Transfusionen dar. Eine gängige Vorstellung in der Fachwelt ist, dass eine theoretische vollständige Verwechslungssicherheit nur durch eine 100% unterbrechungsfreie, in das Krankenhaus integrierte, elektronische Überwachung aller Einzel-Komponenten (Patientenidentität, Laborproben, Blutprodukt) von der Blutabnahme der Laborproben bis zur Transfusion am Patientenbett gewährleistet werden kann. Systeme, welche dies leisten, haben aber den Nachteil, dass sie einen erheblichen Hardware- und Softwareeinsatz erfordern, den sich viele Krankenhäuser nicht leisten können. Hinzu kommen erhebliche personal- und zeitaufwändige Installationsaufwendungen zur Integration in die krankenhausinternen Datenverarbeitungsstrukturen und erforderliche Bereitstellung in allen Bereichen des Krankenhauses, in welchen Blut transfundiert werden soll.

Die vorliegende Erfindung geht einen radikal anderen und deutlich einfacheren Weg. Zunächst wird entgegen der gängigen Vorstellung davon abgesehen, die bestehenden und technisch bereits etablierten partiell-elektronischen Dokumentations- und Kontrollsysteme mit verschiedenen Nummernkreisläufen, Software- und Hardwaremodulen in der Infrastruktur des Krankenhauses weiter zu entwickeln oder zu perfektionieren. Vorliegend wird also der Missstand akzeptiert, dass es in Krankenhäusern unterschiedlichste, nicht perfekte, technische Prozessabläufe und Sicherheitsketten/-strukturen gibt. Anstelle des Ansatzes eines voll-elektronischem Systems für das gesamte Krankenhaus zur lückenlosen Überwachung basiert die vorliegende Erfindung auf einer Untersuchung, welche der am Patientenbett verfügbaren Informationen mindestens notwendig sind, um das Verwechslungsproblem zu lösen. Dabei wurde erkannt, dass es zwar unterschiedlichste Datenverarbeitungsstrategien und Prozessabläufe gibt, dass aber am Ende der der Kette, d.h. am Patientenbett, typischerweise redundante Informationen verfügbar sind, welche ein Arzt dann visuell verarbeitet. Entsprechend liegt die Erfindung insbesondere darin, dass die am Patientenbett vorliegenden Informationen elektronisch assistiert, vorzugsweise auch kamerabasiert, durch ein unabhängiges, externes System, nämlich das Endgerät, ausgewertet werden. Diese Lösung ist besonders einfach und kostengünstig. Das Verwechselungsproblem wird demnach erfindungsgemäß dadurch gelöst, dass die nicht perfekten, sehr heterogenen technischen Krankenhauslösungen akzeptiert und nur mit einer minimalistischen externen, digitalen und vorzugsweise kamerabasierten Lösung kombiniert werden, welche lediglich am letzten Schritt der Sicherheitskette (am Patientenbett) die dort insbesondere visuell vorhandenen Informationen (Produktdaten, Patientendaten) verarbeitet. Die hier beschriebene Lösung funktioniert unabhängig von jeglichen im Krankenhaus bereits installierten technischen Systemen und wertet lediglich die vor Ort insbesondere visuell verfügbaren Informationen am Patientenbett aus.

Ein Vorteil der Erfindung ist somit insbesondere, dass auf Schnittstellen zu krankenhausinterner Hardware, Software und Nummernkreisläufe verzichtet wird. Grundsätzlich ist zwar denkbar, dass das Endgerät in anderem Zusammenhang auf eine solche Schnittstelle zugreift, für die hier beschriebene Erfindung ist dies aber nicht erforderlich und findet entsprechend auch nicht statt. Weitere Vorteile sind, dass kein Drucker benötigt wird und die vorliegend beschriebene technische Lösung besonders einfach ist und lediglich ein einzelnes Endgerät erfordert. Die hier beschriebene technische Lösung ist auch mit unterschiedlichsten bestehenden elektronischen Krankenhauslösungen grundsätzlich einfach kombinierbar, ohne diese zu verändern.

Die Produktdaten und die Patientendaten enthalten jeweils wenigsten eine Angabe zu einer Eigenschaft des Blutprodukts beziehungsweise des Patienten. Die Produktdaten sind in dem bereits beschriebenen Kennzeichnungselement enthalten und werden diesem entnommen, z.B. mittels der bereits genannten Leseeinheit. Die Produktdaten enthalten insbesondere einen oder mehrere der folgenden Parameter:
a) Produkt-ID (z.B. Blutproduktnummer),
b) Produkt-Blutgruppe (z.B. 0 RHD pos)
c) Produkttyp (z.B. Erythrozytenkonzentrat)
d) Haltbarkeitsdatum

Zweckmäßigerweise prüft das Endgerät auch, ob das Blutprodukt noch haltbar ist, ob also das aktuelle Datum (d.h. Ist-Datum, auch "heute"), an welchem die Prüfung erfolgt und an welchem die Transfusion erfolgen soll, noch vor dem Haltbarkeitsdatum liegt oder diesem entspricht. Ist das nicht der Fall, wird eine negative Meldung ausgegeben (Blutprodukt ist abgelaufen), ansonsten wird eine positive Meldung ausgegeben, sofern auch alle anderen Prüfungen positiv sind.

Die Patientendaten enthalten personenbezogene Daten des Patienten, welche von einem Identifikationselement bereitgestellt werden, welches dem Patienten zugeordnet ist, oder welche vom Endgerät als manuelle Eingabe z.B. des Arztes erfasst werden. Das Identifikationselement ist allgemein ein Datenträger, vorzugsweise ein einfaches Patientenarmband oder ähnliches, welches vom Patienten getragen wird und auf welchem personenbezogene Daten aufgedruckt sind. Personenbezogene Daten sind insbesondere die folgenden Parameter:
(1) Name des Patienten
(2) Geburtsdatum des Patienten
(3) Patienten-Blutgruppe (Blutgruppe des Patienten)
(4) Fallnummer des Patienten

Insbesondere die Fallnummer ist vorteilhafterweise eindeutig und nur einmal für einen einzigen Patienten vergeben. Die personenbezogenen Daten sind in maschinenlesbarer und/oder menschenlesbarer Form ausgebildet, z.B. sind Name, Geburtsdatum und Fallnummer als Text aufgedruckt und die Fallnummer nochmals redundant in Form eines Barcodes.

Dem Blutprodukt ist zweckmäßigerweise ein Begleitdokument zugeordnet (d.h. korrekt oder fälschlich zugeordnet), welches separat von dem Blutprodukt ausgebildet ist. Das Begleitdokument ist allgemein ein Datenträger, z.B. ein Blatt Papier oder Bildschirm, auf welchem die Daten angezeigt sind. Das Begleitdokument ist höchstens lösbar, in jedem Fall aber nicht fest, mit dem Blutprodukt verbunden und kann auch separat vom Blutprodukt aufbewahrt werden. Zur Vorbereitung der Transfusion werden das Begleitdokument und das Blutprodukt am Patientenbett bereitgestellt. Das Begleitdokument enthält Produktdaten zu einem Blutprodukt und Patientendaten zu einem Patienten als Begleitdaten, um ein Blutprodukt einem Patienten zuzuordnen. Bei falscher Zuordnung enthält das Begleitdokument Produktdaten zu einem anderen Blutprodukt, als dem am Patientenbett bereitgestellten Blutprodukt. In einer vorteilhaften Ausgestaltung erfasst das Endgerät die Begleitdaten (analog zur Beschreibung der Erfassung der Produkt- und Patientendaten) und prüft, ob diese zu den Produktdaten, d.h. den Produktdaten in dem Kennzeichnungselement, und den Patientendaten, d.h. den Patientendaten in dem Identifikationselement oder in einer manuellen Eingabe, passen. Auf diese Weise wird insbesondere auch geprüft, ob dem Patienten vorab das korrekte Blutprodukt zugeordnet wurde.

Das Begleitdokument enthält insbesondere eine Kombination der oben genannten Parameter, d.h. einen oder mehrere der folgenden Parameter:
(1) Produkt-ID (z.B. Blutproduktnummer),
(2) Produkt-Blutgruppe (z.B. 0 RHD pos)
(3) Produkttyp (z.B. Erythrozytenkonzentrat)
(4) Name des Patienten
(5) Geburtsdatum des Patienten
(6) Patienten-Blutgruppe (Blutgruppe des Patienten)
(7) Fallnummer des Patienten oder äquivalent eine andere eindeutige Patienten-ID

Dabei enthält das Begleitdokument immer wenigstens einen Parameter aus den Produktdaten und einen Parameter aus den Patientendaten, um eine Zuordnung zwischen Blutprodukt und Patient herzustellen. Welche Parameter genaue in dem Begleitdokument enthalten sind, kann auch für verschiedene Blutprodukte unterschiedliche sein. Insbesondere enthält das Begleitdokument im Falle eines Erythrozytenkonzentrats auch ein Gültigkeitsdatum einer Verträglichkeitsprobe, welche vorab durchgeführt worden ist. Das Endgerät prüft dann zweckmäßigerweise analog zum oben erwähnten Haltbarkeitsdatum, ob die Verträglichkeitsprobe noch gültig ist, ob also das aktuelle Datum (d.h. Ist-Datum, auch "heute"), an welchem die Prüfung erfolgt und an welchem die Transfusion erfolgen soll, noch vor dem Gültigkeitsdatum liegt oder diesem entspricht. Ist das nicht der Fall, wird eine negative Meldung ausgegeben (Verträglichkeitsprobe ist ungültig), ansonsten wird eine positive Meldung ausgegeben, sofern auch alle anderen Prüfungen positiv sind.

Das Endgerät prüft nun einen oder mehrere der folgenden Fälle, d.h. die oben genannte Prüfung, ob die Produktdaten und die Patientendaten zueinander passen, umfasst einen oder mehrere der folgenden Fälle:
a. ob eine Patienten-ID in den Begleitdaten mit einer Patienten-ID in den Patientendaten übereinstimmt. Dies entspricht insbesondere einer Identitätsprüfung. Dabei ist die Patienten-ID vorzugsweise ein Name, ein Geburtsdatum, eine Fallnummer oder eine Kombination davon.
b. ob eine Produkt-ID, z.B. Blutkonservennummer, in den Produktdaten mit einer Produkt-ID in den Begleitdaten übereinstimmt. Damit wird insbesondere geprüft, ob das Blutprodukt und das Begleitdokument tatsächlich zusammengehören.
c. ob eine Produkt-Blutgruppe in den Produktdaten mit einer Produkt-Blutgruppe in den Begleitdaten kompatibel ist, vorzugsweise übereinstimmt.
d. ob eine Patienten-Blutgruppe in den Patientendaten mit einer Patienten-Blutgruppe in den Begleitdaten kompatibel ist, vorzugsweise übereinstimmt.
e. ob eine Patienten-Blutgruppe in den Begleitdaten mit einem Ergebnis eines Bluttests, insbesondere ABO-Bedside-Tests, kompatibel ist, vorzugsweise übereinstimmt.

Dabei sind generell mit "Patientendaten" und "Produktdaten" nicht diejenigen gemeint, welche in den Begleitdaten enthalten sind, sondern diejenigen, welche in anderen Datenquellen enthalten sind, insbesondere in dem Kennzeichnungselement, dem Identifikationselement und einer manuellen Eingabe. Die Prüfung, ob die Produktdaten und die Patientendaten zueinander passen, erfolgt somit indirekt über das Begleitdokument. Der Begriff Patienten-ID wird daher vorliegend als Oberbegriff für diese Parameter verwendet, sowohl für jeden einzelnen Parameter als auch für Kombinationen mehrerer dieser Parameter. Die Patienten-ID wird also aus einem oder mehreren der Parameter Name, Geburtsdatum und Fallnummer gebildet. Die Patienten-Blutgruppe in den Begleitdaten wurde insbesondere im Vorfeld mittels eines Bluttests ermittelt, um das zu transfundierende Blutprodukt zu ermitteln. Das Ergebnis dieses Bluttests kann auch in die Patientendaten aufgenommen werden, vorzugsweise ist die Patienten-Blutgruppe in den Patientendaten jedoch das Ergebnis eines weiteren, separaten Tests, insbesondere eines AB0-Test, welcher direkt am Patientenbett und erst kurz vor der Transfusion vom transfundierenden Arzt durchgeführt wird.

Alternativ oder zusätzlich erfolgt die Prüfung direkt, d.h. wenigstens ein Parameter der Produktdaten aus dem Kennzeichnungselement wird mit wenigstens einem Parameter der Patientendaten abgeglichen und anhand dessen wird ermittelt, ob Produktdaten und Patientendaten zueinander passen. In einer bevorzugten Ausgestaltung enthalten die Patientendaten eine Patienten-Blutgruppe, welche in einem Identifikationselement enthalten ist, welches dem Patienten zugeordnet ist, und das Endgerät prüft, ob eine Produkt-Blutgruppe in den Produktdaten mit der Patienten-Blutgruppe kompatibel ist, d.h. effektiv ob das Blutprodukt und der Patient kompatible Blutgruppen aufweisen. Die Prüfung, ob die Produktdaten und die Patientendaten zueinander passen, umfasst demnach den Abgleich der Blutgruppen von Blutprodukt und Patient.

Zweckmäßigerweise enthalten die Patientendaten das Ergebnis eines Bluttests, insbesondere AB0-Bedside-Tests. Auch hier sind mit "Patientendaten" nicht diejenigen gemeint, welche in den Begleitdaten enthalten sind, sondern diejenigen, welche in anderen Datenquellen enthalten sind, insbesondere in dem Kennzeichnungselement, dem Identifikationselement und einer manuellen Eingabe. Die Durchführung des Bluttests ist nicht Teil des Verfahrens, sondern wird zuvor oder parallel dazu vom Arzt durchgeführt, aber ebenfalls am Patientenbett als sogenannter Bedside-Test. Der Bluttest liefert eine Blutgruppe, genauer Patienten-Blutgruppe, welche dann Teil der Patientendaten ist und mit der Produkt-Blutgruppe des Kennzeichnungselements und/oder des Begleitdokuments abgleichbar ist. Entsprechend prüft das Endgerät in einer geeigneten Ausgestaltung, ob das Ergebnis des Bluttests mit einer Produkt-Blutgruppe in den Produktdaten kompatibel ist. Alternativ oder zusätzlich prüft das Endgerät geeigneterweise, ob das Ergebnis des Bluttests mit einer Produkt-Blutgruppe in den Patientendaten kompatibel ist.

Die Prüfung, ob die Produktdaten und die Patientendaten zueinander passen, umfasst demnach den Abgleich der Blutgruppen von Blutprodukt und Patient, wobei die Blutgruppe des Patienten durch einen AB0-Bedside-Test vorgegeben ist, welcher vom Arzt durchgeführt wurde. Das Ergebnis des Bluttests wird vorzugsweise als eine manuelle Eingabe mittels eines Eingabeelements des Endgeräts erfasst. Alternativ ist auch eine Erfassung des Bluttests mittels der optischen Leseeinheit möglich, mit einer anschließenden Bestimmung des Ergebnisses und somit der Blutgruppe durch das Endgerät mittels einer Bilderkennung des Endgeräts.

Beim Abgleich zweier Blutgruppen wie oben verschiedentlich beschrieben, ist es für eine positive Meldung grundsätzlich nicht zwingend erforderlich, dass diese übereinstimmen, also identisch sind, sondern es ist ausreichend, wenn die miteinander verglichenen Blutgruppen kompatibel sind. Zumindest gilt dies für den Abgleich der Patienten-Blutgruppe mit der Produkt-Blutgruppe. Für den Abgleich des Ergebnisses des Bluttestes, welchen der Arzt zur Bestimmung der Patienten-Blutgruppe selbst am Patientenbett durchführt (Blutgruppenbestimmung am Krankenbett), gilt hingegen vorzugsweise, dass hier die beiden Blutgruppen gleich sein müssen, d.h. das Endgerät prüft, ob das Ergebnis des Bluttests mit der Patienten-Blutgruppe identisch ist, welche in dem Begleitdokument enthalten ist.

Um zu beurteilen, ob zwei miteinander verglichene Blutgruppen kompatibel sind, wird insbesondere eine Beurteilungsvorschrift verwendet, welche in dem Endgerät gespeichert ist, z.B. als Teil des Computerprogramms. Die Beurteilungsvorschrift erhält als Eingangsparameter die beiden abzugleichenden Blutgruppen und gibt dann aus, ob diese kompatibel sind oder nicht. Damit wird der Tatsache Rechnung getragen, dass bei manchen Blutprodukten auch unterschiedliche Blutgruppen miteinander kompatibel sein können. Beispielsweise kann unabhängig von der Blutgruppe des Patienten ein Frischplasma der Blutgruppe AB verabreicht werden oder ein Erythrozytenkonzentrat der Blutgruppe 0, sodass die Beurteilungsvorschrift in diesen beiden Fällen für alle Patienten-Blutgruppen ausgibt, dass diese kompatibel mit der Produkt-Blutgruppe sind. Entsprechend wird zweckmäßigerweise beim Abgleich zweier Blutgruppen auch ein Produkttyp des Blutprodukts berücksichtigt. Der Produkttyp ist insbesondere in den Produktdaten enthalten. Das Endgerät prüft dann also, ob die beiden Blutgruppen in Anbetracht des Produkttyps kompatibel sind.

Grundsätzlich wird eine positive Meldung nur dann ausgegeben, wenn alle durchgeführten Prüfungen eine Übereinstimmung oder Kompatibilität ergeben. Bei einer einzelnen Abweichung wird hingegen eine negative Meldung ausgegeben. Die jeweilige Meldung wird über ein Ausgabeelement des Endgeräts ausgegeben, z.B. ein Touchdisplay, normales Display oder einen Lautsprecher.

Ein Vorteil der Erfindung ist insbesondere auch, dass diese in unterschiedlichsten Informationssituationen funktioniert, d.h. dass auch bei teilweise fehlenden Daten wenigstens noch eine Prüfung im Rahmen der verfügbaren und erfassten Daten erfolgt. Beispielsweise ist es möglich, dass jegliche konkreten Patientendaten fehlen, z.B. im Vorfeld einer Soforttransfusion in einer Notfallsituation. Als Patientendaten wird also lediglich angegeben, dass diese unbekannt sind. Bei einem anderen Beispiel fehlen die Begleitdokumente oder das Ergebnis eines AB0-Bedside-Tests fehlt. In diesem Fall verwendet das Endgerät je nachdem, welche Daten verfügbar sind, gegebenenfalls andere Bewertungskriterien bei der Prüfung, ob die Produktdaten und die Patientendaten zueinander passen. Beispielsweise ist es grundsätzlich bereits ausreichend, einerseits die Produkt-ID in dem Kennzeichnungselement und in dem Begleitdokument abzugleichen und andererseits die Fallnummer in dem Identifikationselement und in dem Begleitdokument. Ist allerdings auf Grundlage der erfassten Daten überhaupt keine Prüfung möglich, dann gibt das Endgerät einen entsprechenden Hinweis und/oder eine negative Meldung aus.

Speziell im beschriebenen Fall, dass keinerlei Patientendaten vorliegen und somit speziell die Patienten-Blutgruppe unbekannt ist, weist das Endgerät zweckmäßigerweise einen Notfallmodus auf, in welchem zumindest geprüft wird, ob die Produkt-Blutgruppe allgemeinverträglich ist, d.h. kompatibel unabhängig von der Patienten-Blutgruppe. Insbesondere enthalten die Produktdaten dann eine Produkt-Blutgruppe und einen Produkttyp und als eine Patienten-Blutgruppe ist angegeben, dass diese unbekannt ist (Patienten-Blutgruppe = unbekannt). In diesem Fall schaltet das Endgerät dann geeigneterweise in einen Notfallmodus um, in welchem das Endgerät die Produktdaten erfasst und prüft, ob in Anbetracht des Produkttyps und der Produkt-Blutgruppe das Blutprodukt allgemeinverträglich ist. Das Ausgeben einer positiven/negativen Meldung erfolgt dann analog zur bereits beschriebenen regulären Prüfung.

Nachfolgend werden zur Verdeutlichung der Erfindung drei konkrete Anwendungssituationen für die Erfindung beschrieben. Die Erfindung ist nicht auf diese drei Anwendungssituationen und die im Zusammenhang damit genannten Merkmalskombinationen beschränkt. Auch sind Teilmengen der Merkmale verschiedener Anwendungssituationen grundsätzlich miteinander kombinierbar.

Für alle drei Anwendungssituationen ist das Endgerät ein kamerabasiertes, mobiles Endgerät, d.h. ein mobiles Endgerät mit einer integrierten Kamera, z.B. Smartphone, Tablet, PDA oder dergleichen. Insbesondere kann dasselbe Endgerät für alle drei Anwendungssituationen genutzt werden. Das Endgerät weist die folgenden technischen Eigenschaften auf:
(1) Kamerabasierte Texterkennung
(2) Kamerabasierte Codeerkennung (Barcode-Lesefähigkeit)
(3) Programmierbarer Datenabgleich
(4) Menügesteuerte Nutzerführung
(5) Beurteilung ABO Verträglichkeit

Das Endgerät liest visuelle Informationen, welche am Patientenbett entweder in Text- oder Codeform (Barcode) vorliegen ein, d.h. erfasst Produktdaten und Patientendaten, und vergleicht diese automatisch miteinander auf Übereinstimmung und Integrität, d.h. prüft, die Produktdaten zu den Patientendaten passen.

Im ersten Ausführungsbeispiel werden die folgenden Parameter kamerabasiert eingelesen und verarbeitet:
(1) Identifikationselement, speziell Patientenarmband (Name, Geburtsdatum; Barcodekennung, sofern vorhanden)
(2) Blutprodukt (Produkt-ID als Barcode, Produkt-Blutgruppe als Barcode)
(3) Begleitdokument (Name und Geburtsdatum des Patienten; Produkt-ID des ausgewählten Blutprodukts als Text oder Barcode, Patienten-Blutgruppe)

Optional wird zusätzlich noch das Ergebnis eines AB0-Bedside-Tests mittels einer manuellen Eingabe des Arztes, welcher den Test durchgeführt hat, erfasst (Blutgruppenbestimmung am Krankenbett).

Das Endgerät vergleicht nun die Patientendaten, darin auch optional das Ergebnis des ABO-Bedside-Tests, die Blutproduktdaten und die Begleitdaten miteinander auf Übereinstimmung und generiert eine Transfusionsempfehlung in Form einer positiven oder negativen Meldung. Optional wird das Ergebnis dokumentiert.

In einer Notfallsituation oder in Ländern außerhalb Deutschlands kann es sein, dass nur ein Teil der genannten Parameter am Krankenbett visuell vorliegt. In diesen Fällen generiert das Endgerät ebenfalls eine Transfusionsempfehlung, jedoch immer nur aufgrund der vorliegenden visuellen Informationen am Krankenbett.

Im zweiten Ausführungsbeispiel soll eine lebensrettende Notfalltransfusion erfolgen, die Patienten-Blutgruppe ist unbekannt, der Patient ist namenlos. Das Endgerät erfasst Parameter vom Patientenarmband, sofern vorhanden, und jedenfalls von dem Blutprodukt (Produkt-ID als Barcode, Produkt-Blutgruppe als Barcode). Anschließend generiert das Endgerät eine Transfusionsempfehlung und dokumentiert (optional) das Ergebnis. Bei Patienten mit unbekannter Blutgruppe dürfen beispielsweise nur Erythrozytenkonzentrate der Blutgruppe 0 und Frischplasma der Blutgruppe AB verabreicht werden. In diesem Ausführungsbeispiel wird somit eine Verwechslung mit einer AB0-unverträglichen Blutkonserve verhindert.

Im dritten Ausführungsbeispiel soll eine Transfusion in einem Land ohne vorgeschriebenen AB0-Bedside-Test erfolgen, in den Patientendaten fehlt also das Ergebnis eines solchen AB0-Bedside-Test. Die folgenden Parameter werden vom Endgerät kamerabasiert eingelesen und verarbeitet:
(1) Identifikationselement, speziell Patientenarmband (Name, Geburtsdatum; Barcodekennung, sofern vorhanden)
(2) Blutprodukt (Produkt-ID als Barcode, Produkt-Blutgruppe als Barcode)
(3) Begleitdokument (Name und Geburtsdatum des Patienten; Produkt-ID des ausgewählten Blutprodukts als Text oder Barcode, Patienten-Blutgruppe)

Das Endgerät generiert dann eine Transfusionsempfehlung und dokumentiert (optional) das Ergebnis. In diesem Ausführungsbeispiel wird somit einerseits eine Verwechslung mit einem anderen als auf dem Begleitdokument angegebenen Blutprodukt verhindert sowie andererseits eine Verwechslung mit einem anderen als auf dem Begleitdokument angegebenen Patienten.

Ein erfindungsgemäßes Endgerät ist ausgebildet, ein Verfahren wie oben beschrieben auszuführen.

Ein erfindungsgemäßes Computerprogramm weist Befehle auf, welche bewirken, dass ein Endgerät wie beschrieben die Verfahrensschritte eines Verfahrens wie beschrieben ausführt. Insbesondere sind die diversen oben beschriebenen Verfahrensschritte, in welchen das Endgerät eine Übereinstimmung oder Kompatibilität zweier Parameter miteinander prüft, mit dem Computerprogramm realisiert. Im Kern erfolgt dabei bei jeder einzelnen Prüfung insbesondere ein Vergleich zweier Parameter aus unterschiedlichen Datenquellen. Dabei sind die Datenquellen a) die verschiedenen Datenträger, nämlich Kennzeichnungselement, Begleitdokument, Identifikationselement, von welchen Daten direkt, z.B. mittels der Leseeinheit erfasst werden oder indirekt mittels manueller Eingabe über das Eingabeelement des Endgeräts, und b) generell manuelle Eingaben von über das Eingabeelement des Endgeräts von solchen Daten, welche nicht einem der vorgenannten Datenträger enthalten sind, wie z.B. das bereist beschriebene Ergebnis eines unmittelbar vor der Transfusion am Patientenbett durchgeführten Bluttests. Im einfachsten Fall ist der Vergleich eine einfache Gleichheitsprüfung, d.h. ob die beiden Parameter übereinstimmen, d.h. identisch sind. Dies ist z.B. der Fall für die Patienten-ID oder die Produkt-ID. Bei manchen Parametern, wie z.B. der Blutgruppe, kann der Vergleich zwar grundsätzlich auch eine solche Gleichheitsprüfung sein, alternativ ist der Vergleich aber eine Kompatibilitätsprüfung, wie oben bereits beschrieben. Bei einer solchen Kompatibilitätsprüfung wird z.B. eine Kompatibilitätsdatenbank verwendet, welche für alle möglichen Paarungen von Werten der zwei Parameter, welche miteinander verglichen werden, angibt, ob diese für eine jeweilige Paarung von Werten kompatibel sind oder nicht. Dabei werden - wie beschrieben - gegebenenfalls noch weitere Parameter berücksichtigt, z.B. im Falle der Blutgruppe der Produkttyp. Die Kompatibilitätsdatenbank ist vorzugsweise auf dem Endgerät gespeichert und/oder ein Teil des Computerprogramms. Ganz allgemein ist vorzugsweise das gesamte Computerprogramm in einem Speicher des Endgeräts gespeichert, um eine stand-alone-Lösung zu realisieren.

Nachfolgend werden Ausführungsbeispiele der Erfindung anhand einer Zeichnung näher erläutert. Darin zeigen jeweils schematisch:
Fig. 1 ein Verfahren,
Fig. 2 eine Anwendungssituation,
Fig. 3 ein Kennzeichnungselement eines Blutprodukts,
Fig. 4 ein Begleitdokument
Fig. 5 ein Endgerät in einer Frontansicht und mit positiver Meldung,
Fig. 6 das Endgerät aus Fig. 5 in einer Seitenansicht,
Fig. 7 das Endgerät aus Fig. 5 mit einer negativen Meldung,
Fig. 8 ein Identifikationselement,
Fig. 9 einen Bluttest.

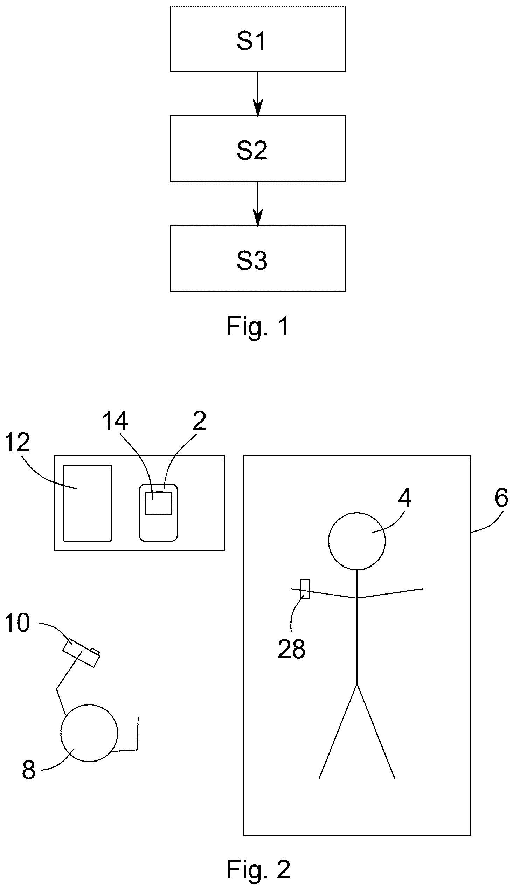

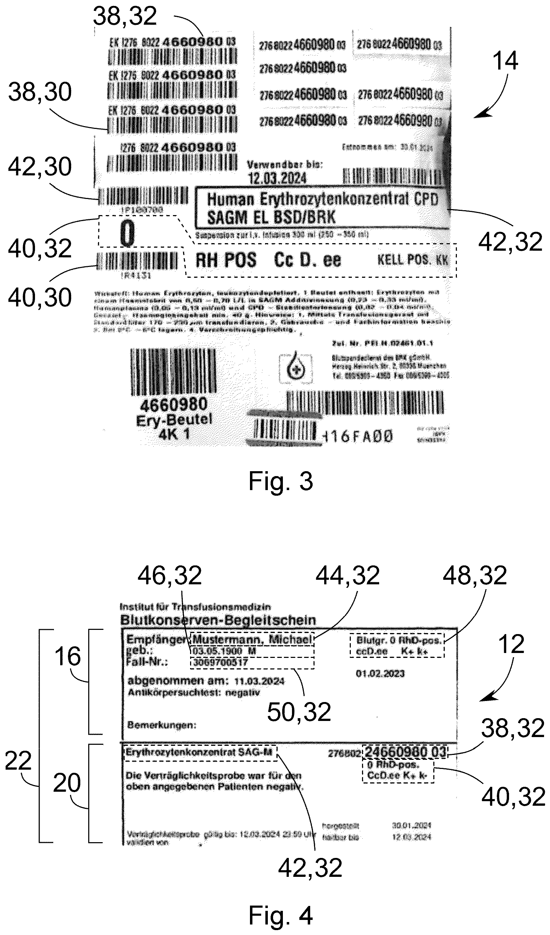

Fig. 1 zeigt ein Ausführungsbeispiel des erfindungsgemäßen Verfahrens. Das gezeigte Verfahren dient zur Prüfung einer Zuordnung eines Blutprodukts 2 zu einem Patienten 4. Eine Anwendungssituation für das Verfahren ist in Fig. 2 gezeigt, darin sind in einer Ansicht von oben gezeigt: der Patient 4 in einem Patientenbett 6, ein Arzt 8 mit einem Endgerät 10 sowie das Blutprodukt 2 und ein Begleitdokument 12, welche gemeinsam auf einer nicht explizit bezeichneten Ablagefläche bereitgestellt sind. Das Blutprodukt 2 ist mit einem Kennzeichnungselement 14 gekennzeichnet, welches Produktdaten 16 des Blutprodukts 2 enthält. Ein beispielhaftes Kennzeichnungselement 14 ist im Detail in Fig. 3 gezeigt. Das Blutprodukt 2 ist vorliegend in einem Beutel aufbewahrt und das Kennzeichnungselement 14 ist ein Etikett, welches auf den Beutel aufgeklebt ist und somit dem Blutprodukt 2 fest zugeordnet ist. Ein beispielhaftes Begleitdokument 12 ist in Fig. 4 gezeigt. Das Begleitdokument 12 enthält Begleitdaten 22.

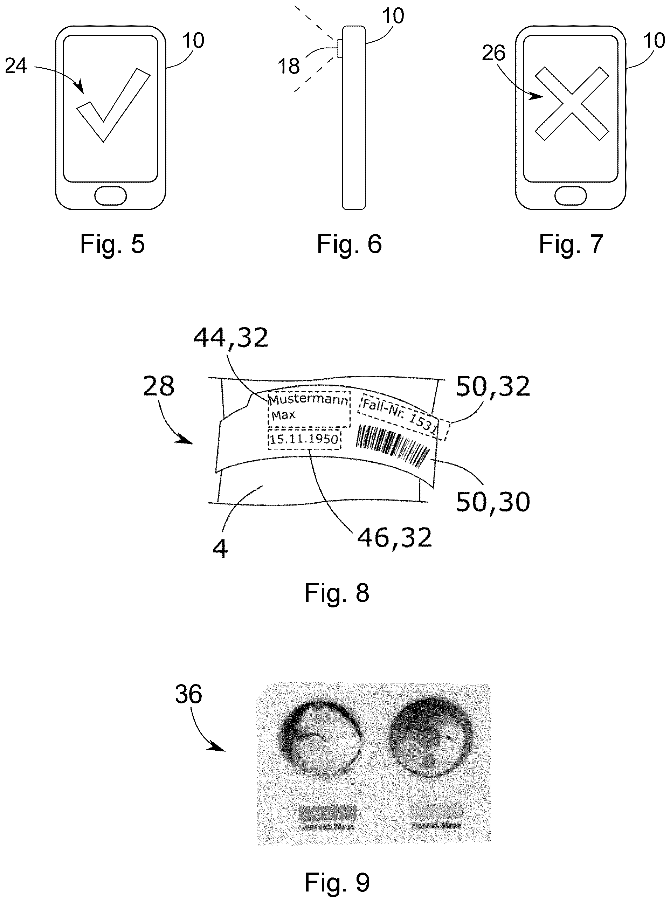

Ein beispielhaftes Endgerät 10 ist in einer Frontansicht in Fig. 5 gezeigt und in einer Seitenansicht in Fig. 6. Das hier gezeigte Endgerät 10 ist ein mobiles Endgerät, speziell ein Smartphone. Das Endgerät weist auch eine optische Leseeinheit 18 auf, hier eine Kamera, deren Blickfeld in Fig. 6 durch zwei Strichlinien dargestellt ist.

Das Endgerät 10 erfasst nun in einem ersten Verfahrensschritt S1 sowohl die Produktdaten 16 als auch Patientendaten 20. In einem zweiten Verfahrensschritt S2 prüft das Endgerät 10, ob die Produktdaten 16 und die Patientendaten 20 zueinander passen. In einem dritten Verfahrensschritt S3 gibt das Endgerät 10 eine positive Meldung 24 (Prüfung bestanden, Zuordnung korrekt) aus, wenn die Produktdaten 16 zu den Patientendaten 20 passen, und ansonsten eine negative Meldung 26 (Prüfung nicht bestanden, Zuordnung nicht korrekt). Das eigentliche Verfahren wird somit von dem Endgerät 10 ausgeführt. Ein Beispiel für das Ausgeben einer positiven Meldung 24 ist in Fig. 5 gezeigt, ein Beispiel für das Ausgeben einer negativen Meldung 26 ist in Fig. 7 gezeigt, welche auch das gleiche Endgerät 10 wie in den Fig. 5 und 6 zeigt.

Vorliegend ist dem Patienten 4 ein Identifikationselement 28 zugeordnet, hier ein Patientenarmband, welches vom Patienten 4 am Arm getragen wird. Das Identifikationselement 28 enthält Patientendaten 20. In Fig. 8 ist ein Ausführungsbeispiel für das Identifikationselement 28 im Detail gezeigt, hier ein Patientenarmband am Handgelenk des Patienten 4.

Die hier vorgestellte Erfindung ist nicht einfach nur ein automatisiertes Abgleichen der Daten 16, 20, 22, vielmehr erfolgt sowohl das Erfassen und Prüfen der Daten 16, 20, 22 als auch das Ausgeben der Meldung 24, 26 mittels eines einzelnen Endgeräts 10, welches selbstständig arbeitet und unabhängig ist von übrigen Geräten, speziell Krankenhausinfrastruktur. Das Endgerät 10 wird lediglich von einem Bediener, hier dem Arzt 8, jeweils so ausgerichtet und/oder bedient, dass dieses die Daten 16, 20, 22 erfassen kann. Das Endgerät 10 ist somit eine "stand-alone"-Lösung und benötigt für die Erfassung und für die Prüfung keine externe Datenbank und auch kein externes Gerät und greift auf solche auch nicht zu. Das Verfahren und das Endgerät 10 werden wie in Fig. 2 gezeigt in räumlicher Nähe des Patienten 4 eingesetzt. Das Verfahren wird als Abschluss einer Vorbereitung der Transfusion und zeitlich unmittelbar vor dieser Transfusion durchgeführt.

Durch das Endgerät 10 wird ein Arzt 8 bei der Entscheidungsfindung unterstützt, ob das am Patientenbett 6 bereitgestelltes Blutprodukt 2 transfundiert werden kann oder nicht. Das Endgerät 10 wird dabei vom Arzt 8 bedient. Die Unterstützung besteht darin, dass das Endgerät 10 die verschiedenen zu prüfenden Daten 16, 20, 22, erfasst, miteinander abgleicht und dann eine entsprechende Meldung 24, 26 ausgibt. Die Meldung 24, 26 wird auch als "Transfusionsempfehlung" bezeichnet. Im Kern führt das Endgerät 10 somit automatisiert eine Integritätsprüfung der vor Ort vorliegenden Daten 16, 20, 22 durch, womit anhand dieser Daten 16, 20, 22 effektiv geprüft wird, ob dem Patienten 4 letztendlich das korrekte Blutprodukt 2 zur Transfusion zugeordnet wurde. Abhängig vom Ergebnis der Prüfung gibt das Endgerät 10 dann eine entsprechende Meldung 24, 26 aus, welche der Arzt 8 bei der Entscheidung, ob das Blutprodukt 2 transfundiert wird oder nicht, verwenden kann. Die tatsächliche Entscheidung, ob eine Transfusion erfolgt, und auch die tatsächliche Transfusion, werden jedoch vom Arzt 8 vorgenommen, das Endgerät 10 ist hieran nicht beteiligt.

Mit der optischen Leseeinheit 18 erfasst das Endgerät 10 die Produktdaten 16, die Patientendaten 20 und auch die Begleitdaten 22, wobei letztere aus Produktdaten 16 und Patientendaten 20 zusammengesetzt sind. Hierbei wird der jeweilige Datenträger (Kennzeichnungselement 14, Begleitdokument 12, Identifikationselement 28) mit der Leseeinheit 18 gescannt oder fotografiert. Sämtliche Daten 16, 20, 22, welche bei der nachfolgenden Prüfung verwendet werden, werden mittels desselben Endgeräts 10 erfasst. Wie in den Fig. 3, 4 und 8 erkennbar ist, liegen die Daten 16, 20, 22 ganz oder teilweise in Form von einem oder mehreren maschinenlesbaren Codes 30, hier Barcodes, oder von menschenlesbarem Text 32, hier Zeichenketten, vor oder eine Kombination hiervon. Insgesamt sind die Daten 16, 20, 22 generell visuell verfügbar und können daher mit der optischen Leseeinheit 18 erfasst werden.

Grundsätzlich ist es ausreichend, wenn die jeweiligen Daten 16, 20, 22 entweder in Form eines maschinenlesbaren Codes 30 oder in Form von menschenlesbarem Text 32. Vorliegend ist jedoch teilweise beides der Fall, sodass die Produktdaten 16 und die Patientendaten 20 vom Endgerät 10 redundant auf wenigstens zwei verschiedene Weisen erfasst werden. Entsprechend ist dann auch die Prüfung, welche das Endgerät 10 durchführt, redundant.

Zusätzlich weist das hier gezeigte Endgerät 10 ein Eingabeelement 34 auf, hier ein Touchscreen, über welches zumindest ein Teil der Daten 18, 20, 22 manuell eingegeben werden kann und dann als manuelle Eingabe erfasst wird. Dies wird vorliegend genutzt, um das Ergebnis eines Bluttests 36 zur Bestimmung der Patienten-Blutgruppe am Patientenbett 6 (AB0-Bedside-Test). Ein solcher Bluttest 36 ist beispielhaft in Fig. 9 gezeigt. Darin ist erkennbar, dass der Bluttest 36 zwei Felder aufweist, nämlich ein Feld auf der linken Seite, welches mit "Anti-A" beschriftet ist, und ein Feld auf der rechten Seite, welches mit "Anti-B" beschriftet ist. Das Ergebnis wird vom Arzt 8 festgestellt und dann in das Endgerät 10 eingegeben und damit von diesem erfasst. Grundsätzlich können auch alle anderen Daten 18, 20, 22 als manuelle Eingabe erfasst werden.

Die erfassten Daten 16, 20, 22 und/oder das Ergebnis der Prüfung werden gegebenenfalls auch gespeichert (Protokollierungsfunktion), z.B. auf dem Endgerät 10, dies ist aber nicht zwingend erforderlich.

Somit werden insgesamt die am Patientenbett 6 vorliegenden Informationen (Daten 16, 20, 22 und gegebenenfalls Ergebnis des Bluttests 36) elektronisch assistiert und vorliegend auch kamerabasiert durch ein unabhängiges, externes System, nämlich das Endgerät 10, ausgewertet. Das Verwechselungsproblem wird demnach dadurch gelöst, dass die nicht perfekten, sehr heterogenen technischen Krankenhauslösungen akzeptiert und nur mit einer minimalistischen externen, digitalen und hier auch kamerabasierten Lösung kombiniert werden, welche lediglich am letzten Schritt der Sicherheitskette (am Patientenbett 6) die dort visuell vorhandenen Informationen verarbeitet. Diese Lösung funktioniert unabhängig von jeglichen im Krankenhaus bereits installierten technischen Systemen und wertet lediglich die vor Ort visuell verfügbaren Informationen am Patientenbett 6 aus. Auf Schnittstellen zu krankenhausinterner Hardware, Software und Nummernkreisläufe wird verzichtet.

Die Produktdaten 16 und die Patientendaten 20 enthalten jeweils wenigsten eine Angabe zu einer Eigenschaft des Blutprodukts 2 beziehungsweise des Patienten 4. Die Produktdaten 16 sind in dem bereits beschriebenen Kennzeichnungselement 14 enthalten und werden diesem entnommen, z.B. mittels der bereits genannten Leseeinheit 18. Die Produktdaten 16 enthalten einen oder mehrere der folgenden Parameter:
a) Produkt-ID 38 (z.B. Blutproduktnummer),
b) Produkt-Blutgruppe 40 (z.B. 0 RHD pos)
c) Produkttyp 42 (z.B. Erythrozytenkonzentrat)
d) Haltbarkeitsdatum

Die Patientendaten 20 enthalten personenbezogene Daten des Patienten 4, welche von dem Identifikationselement 28 bereitgestellt werden und/oder vom Endgerät 10 als manuelle Eingabe des Arztes 8 erfasst werden. Eine manuelle Eingabe erfolgt vorliegend im Falle des Ergebnisses des Bluttests 36. Personenbezogene Daten sind die folgenden Parameter:
(1) Name des Patienten 44
(2) Geburtsdatum des Patienten 46
(3) Patienten-Blutgruppe 48 (Blutgruppe des Patienten)
(4) Fallnummer des Patienten 50 (oder andere eindeutige Patienten-ID)

Die Fallnummer 50 ist eindeutig und nur einmal für einen einzigen Patienten 4 vergeben. Die personenbezogenen Daten sind in maschinenlesbarer und/oder menschenlesbarer Form ausgebildet, z.B. sind wie im Beispiel der Fig. 9 Name 44, Geburtsdatum 46 und Fallnummer 50 als Text aufgedruckt und die Fallnummer 50 nochmals redundant in Form eines Barcodes. Aus Name 44, Geburtsdatum 46 und Fallnummer 50 wird auch eine Patienten-ID 44, 46, 50 gebildet.

Dem Blutprodukt 2 ist vorliegend auch noch das bereits erwähnte Begleitdokument 12 zugeordnet (d.h. korrekt oder fälschlich zugeordnet). Dieses ist separat von dem Blutprodukt 2 ausgebildet und ist allgemein ein Datenträger, hier ein Blatt Papier, auf welchem die Daten 22 angezeigt, hier aufgedruckt, sind. Alternativ ist der Datenträger ein Bildschirm, auf welchem die Daten 22 angezeigt sind (nicht gezeigt). Das Begleitdokument 12 ist höchstens lösbar, in jedem Fall aber nicht fest, mit dem Blutprodukt 2 verbunden und kann auch separat vom Blutprodukt 2 aufbewahrt werden. Zur Vorbereitung der Transfusion werden das Begleitdokument 12 und das Blutprodukt 2 am Patientenbett 6 bereitgestellt, z.B. wie in Fig. 2 gezeigt. Das Begleitdokument 12 enthält Produktdaten 16 zu einem Blutprodukt 2 und Patientendaten 20 zu einem Patienten 4 als Begleitdaten 22, um ein Blutprodukt 2 einem Patienten 4 zuzuordnen. Das Endgerät 10 erfasst nun auch die Begleitdaten 22 und prüft, ob diese zu den Produktdaten 16 im Kennzeichnungselement 14 und den Patientendaten 20 im Identifikationselement 28 und gegebenenfalls auch zum Ergebnis des Bluttests 36 passen.

Das Begleitdokument 12 enthält eine Kombination der oben genannten Parameter, d.h. eine Kombination von Produktdaten 16 und Patientendaten 20. Im hier gezeigten Beispiel enthält das Begleitdokument 12 auch eine Patienten-Blutgruppe 48, welche vorab für die Auswahl des Blutprodukts 2 bestimmt wurde, d.h. nicht das Ergebnis des am Patientenbett 6 in Vorbereitung der Transfusion durchgeführten Bluttest 36, sondern eines zuvor schon durchgeführten, anderen Bluttests. Grundsätzlich enthält das Begleitdokument 12 immer wenigstens einen Parameter aus den Produktdaten 16 und einen Parameter aus den Patientendaten 20, um eine Zuordnung zwischen Blutprodukt 2 und Patient 4 herzustellen. In Fig. 4 sind alle Daten 16, 20, 22 nur als Text enthalten.

Das Endgerät 10 prüft nun einen oder mehrere der folgenden Fälle:
a. ob die Patienten-ID 44, 46, 50 in den Begleitdaten 22 mit der Patienten-ID 44, 46, 50 in den Patientendaten 20 übereinstimmt.
b. ob die Produkt-ID 38 in den Produktdaten 16 mit der Produkt-ID 38 in den Begleitdaten 12 übereinstimmt.
c. ob die Produkt-Blutgruppe 40 in den Produktdaten 16 mit der Produkt-Blutgruppe 40 in den Begleitdaten 12 kompatibel ist oder sogar übereinstimmt.
d. ob die Patienten-Blutgruppe 48 in den Patientendaten 20 mit der Patienten-Blutgruppe 48 in den Begleitdaten 12 kompatibel ist oder sogar übereinstimmt.
e. ob die Patienten-Blutgruppe 48 in den Begleitdaten 12 mit dem Ergebnis des Bluttests 36 kompatibel ist oder sogar übereinstimmt.

Die Prüfung, ob die Produktdaten 16 und die Patientendaten 20 zueinander passen, erfolgt somit indirekt über das Begleitdokument 12.

Alternativ oder zusätzlich erfolgt die Prüfung direkt, d.h. wenigstens ein Parameter der Produktdaten 16 in dem Kennzeichnungselement 14 wird mit wenigstens einem Parameter der Patientendaten 20 abgeglichen und anhand dessen wird ermittelt, ob Produktdaten 16 und Patientendaten 20 zueinander passen. Dies findet z.B. Anwendung, falls die Patienten-Blutgruppe 48 auch in dem Identifikationselement 28 enthalten ist und das Endgerät 10 dann prüft, ob die Produkt-Blutgruppe 40 in den Produktdaten 16 mit der Patienten-Blutgruppe 48 kompatibel ist, d.h. effektiv ob das Blutprodukt 2 und der Patient 4 kompatible Blutgruppen 40, 48 aufweisen. Kurz gesagt: die Blutgruppen 40, 48 von Blutprodukt 2 und Patient 4 werden abgeglichen.

Wie bereits beschrieben enthalten die Patientendaten 16 optional das Ergebnis eines Bluttests 36, z.B. eines AB0-Bedside-Tests wie in Fig. 9 gezeigt. Der Bluttest 36 liefert die Patienten-Blutgruppe 48, welche dann Teil der Patientendaten 20 ist und mit der Produkt-Blutgruppe 40 des Kennzeichnungselements 14 und/oder des Begleitdokuments 12 abgleichbar ist. Entsprechend prüft das Endgerät 10 dann, ob das Ergebnis des Bluttests 36 mit der Produkt-Blutgruppe 48 in den Produktdaten 16 kompatibel ist. Alternativ oder zusätzlich prüft das Endgerät 10, ob das Ergebnis des Bluttests 36 mit der Produkt-Blutgruppe 48 in den Patientendaten 20 kompatibel ist. Die Prüfung, ob die Produktdaten 16 und die Patientendaten 20 zueinander passen, umfasst demnach den Abgleich der Blutgruppen 40, 48 von Blutprodukt 2 und Patient 4, wobei die Patienten-Blutgruppe 48 durch den Bluttest 36 vorgegeben ist, welcher vom Arzt 8 durchgeführt wurde.

Beim Abgleich zweier Blutgruppen 40, 48 wie oben verschiedentlich beschrieben, ist für eine positive Meldung 24 nicht zwingend erforderlich, dass diese übereinstimmen, also identisch sind, sondern es ist ausreichend, wenn die miteinander verglichenen Blutgruppen 40, 48 kompatibel sind. Um dies zu beurteilen, wird eine Beurteilungsvorschrift verwendet, welche in dem Endgerät 10 gespeichert ist. Die Beurteilungsvorschrift erhält als Eingangsparameter die beiden abzugleichenden Blutgruppen 40, 48 und gibt dann aus, ob diese kompatibel sind oder nicht. Damit wird der Tatsache Rechnung getragen, dass bei manchen Blutprodukten 2 auch unterschiedliche Blutgruppen miteinander kompatibel sein können. Beispielsweise kann unabhängig von der Patienten-Blutgruppe 48 ein Frischplasma der Blutgruppe AB (Produkt-Blutgruppe 40) verabreicht werden oder ein Erythrozytenkonzentrat der Blutgruppe 0 (Produkt-Blutgruppe 40), sodass die Beurteilungsvorschrift in diesen beiden Fällen für alle Patienten-Blutgruppen 48 ausgibt, dass diese kompatibel mit der Produkt-Blutgruppe 40 sind. Entsprechend wird beim Abgleich zweier Blutgruppen 40, 48 auch der Produkttyp 42 des Blutprodukts 2 berücksichtigt. Der Produkttyp 42 ist in den Produktdaten 16 enthalten. Das Endgerät 10 prüft dann also, ob die beiden Blutgruppen 40, 48 in Anbetracht des Produkttyps 42 kompatibel sind.

Grundsätzlich wird eine positive Meldung 24 nur dann ausgegeben, wenn alle durchgeführten Prüfungen eine Übereinstimmung oder Kompatibilität ergeben. Bei einer einzelnen Abweichung wird hingegen eine negative Meldung 26 ausgegeben. Die jeweilige Meldung 24, 26 wird über ein Ausgabeelement 34 des Endgeräts 10 ausgegeben, hier das bereits auch als Eingabeelement 34 verwendete Touchdisplay.

Die Erfindung funktioniert auch in unterschiedlichsten Informationssituationen, d.h. dass auch bei teilweise fehlenden Daten 16, 20, 22, wenigstens noch eine Prüfung im Rahmen der verfügbaren und erfassten Daten 16, 20, 22 erfolgt. Beispielsweise ist es möglich, dass jegliche konkreten Patientendaten 20 fehlen, z.B. im Vorfeld einer Soforttransfusion in einer Notfallsituation. Als Patientendaten 20 wird also lediglich angegeben, dass diese unbekannt sind. Bei einem anderen Beispiel fehlen die Begleitdokumente 12 oder das Ergebnis des Bluttests 36 am Patientenbett 6. In diesem Fall verwendet das Endgerät 10 je nachdem, welche Daten 16, 20, 22 verfügbar sind, gegebenenfalls andere Bewertungskriterien bei der Prüfung, ob die Produktdaten 16 und die Patientendaten 20 zueinander passen. Beispielsweise werden lediglich einerseits die Produkt-ID 38 in dem Kennzeichnungselement 14 und in dem Begleitdokument 12 abgeglichen und andererseits die Fallnummer 50 in dem Identifikationselement 28 und in dem Begleitdokument 12. Ist allerdings auf Grundlage der erfassten Daten 16, 20, 22 überhaupt keine Prüfung möglich, dann gibt das Endgerät 10 einen entsprechenden Hinweis und/oder eine negative Meldung 26 aus.

Speziell in dem Fall, dass keinerlei Patientendaten 20 vorliegen und somit speziell die Patienten-Blutgruppe 48 unbekannt ist, weist das Endgerät 10 optional einen Notfallmodus auf, in welchem zumindest geprüft wird, ob die Produkt-Blutgruppe 40 allgemeinverträglich ist, d.h. kompatibel unabhängig von der Patienten-Blutgruppe 48. Die Produktdaten 16 enthalten dann wenigstens die Produkt-Blutgruppe 40 und den Produkttyp 42 und als Patienten-Blutgruppe 48 ist angegeben, dass diese unbekannt ist (Patienten-Blutgruppe 48 = unbekannt). In diesem Fall schaltet das Endgerät 10 dann in den Notfallmodus um, in welchem das Endgerät 10 die Produktdaten 20 erfasst und prüft, ob in Anbetracht des Produkttyps 42 und der Produkt-Blutgruppe 40 das Blutprodukt 2 allgemeinverträglich ist. Das Ausgeben einer positiven/negativen Meldung 24, 26 erfolgt dann analog zur bereits beschriebenen regulären Prüfung.

### Bezugszeichenliste

- 2: Blutprodukt
- 4: Patient
- 6: Patientenbett
- 8: Arzt
- 10: Endgerät (Smartphone)
- 12: Begleitdokument
- 14: Kennzeichnungselement (Etikett)
- 16: Produktdaten
- 18: optische Leseeinheit (Kamera)
- 20: Patientendaten
- 22: Begleitdaten
- 24: positive Meldung
- 26: negative Meldung
- 28: Identifikationselement (Patientenarmband)
- 30: maschinenlesbarer Code (Barcode)
- 32: menschenlesbarer Text (Zeichenkette)
- 34: Eingabeelement, Ausgabeelement (Touchscreen)
- 36: Bluttest (AB0-Bedside-Test)
- 38: Produkt-ID
- 40: Produkt-Blutgruppe
- 42: Produkttyp
- 44: Name des Patienten
- 46: Geburtsdatum des Patienten
- 48: Patienten-Blutgruppe
- 50: Fallnummer des Patienten
- S1: erster Verfahrensschritt (Erfassung der Daten)
- S2: zweiter Verfahrensschritt (Prüfung der Daten)
- S3: dritter Verfahrensschritt (Ausgeben einer Meldung)

## Patentansprüche

1. Verfahren zur Prüfung einer Zuordnung eines Blutprodukts (2) zu einem Patienten (4),
a. wobei das Blutprodukt (2) mit einem Kennzeichnungselement (14) gekennzeichnet ist, welches Produktdaten (16) des Blutprodukts (2) enthält,
b. wobei ein Endgerät (10)
i. sowohl die Produktdaten (16) erfasst als auch Patientendaten (20),
ii. prüft, ob die Produktdaten (16) und die Patientendaten (20) zueinander passen,
iii. eine positive Meldung (24) ausgibt, wenn die Produktdaten (16) zu den Patientendaten (20) passen, und ansonsten eine negative Meldung (26).

2. Verfahren nach Anspruch 1,
wobei das Endgerät (10) für die Erfassung und für die Prüfung keine externe Datenbank benötigt und auf eine solche auch nicht zugreift und wobei das Endgerät (10) für die Erfassung und für die Prüfung kein externes Gerät benötigt und auf ein solches auch nicht zugreift.

3. Verfahren nach Anspruch 1 oder 2,
wobei das Endgerät (10) eine optische Leseeinheit (18), insbesondere eine Kamera, aufweist, mit welcher die Produktdaten (16) und/oder die Patientendaten (20) erfasst werden.

4. Verfahren nach einem der Ansprüche 1 bis 3,
wobei die Produktdaten (16) und/oder die Patientendaten (20) in Form eines maschinenlesbaren Codes (30) vorliegen, welcher vom Endgerät (10) eingelesen wird, und zugleich auch in Form von menschenlesbarem Text (32), sodass die Produktdaten (16) und/oder Patientendaten (20) vom Endgerät (10) redundant auf wenigstens zwei verschiedene Weisen erfasst werden.

5. Verfahren nach einem der Ansprüche 1 bis 4,
wobei dem Blutprodukt (2) ein Begleitdokument (12) zugeordnet ist, welches separat von dem Blutprodukt (2) ausgebildet ist,
wobei das Begleitdokument (12) Produktdaten (16) zu einem Blutprodukt (2) und Patientendaten (20) als Begleitdaten (22) enthält,
wobei das Endgerät (10) die Begleitdaten (22) erfasst und prüft, ob diese zu den Produktdaten (16) und den Patientendaten (20) passen.

6. Verfahren nach Anspruch 5,
wobei das Endgerät (10) einen oder mehrere der folgenden Fälle prüft:
a. ob eine Patienten-ID (44, 46, 50) in den Begleitdaten (22) mit einer Patienten-ID (44, 46, 50) in den Patientendaten (20) übereinstimmt,
b. ob eine Produkt-ID (38) in den Produktdaten (20) mit einer Produkt-ID (38) in den Begleitdaten (22) übereinstimmt,
c. ob eine Produkt-Blutgruppe (40) in den Produktdaten (20) mit einer Produkt-Blutgruppe (40) in den Begleitdaten (22) kompatibel ist,
d. ob eine Patienten-Blutgruppe (48) in den Patientendaten (16) mit einer Patienten-Blutgruppe (48) in den Begleitdaten (22) kompatibel ist,
e. ob eine Patienten-Blutgruppe (48) in den Begleitdaten (22) mit einem Ergebnis eines Bluttests (36), insbesondere ABO-Bedside-Tests, kompatibel ist.

7. Verfahren nach einem der Ansprüche 1 bis 6,
wobei die Patientendaten (20) das Ergebnis eines Bluttests (36), insbesondere ABO-Bedside-Tests, enthalten,
wobei das Endgerät (10) prüft, ob das Ergebnis des Bluttests (36) mit einer Produkt-Blutgruppe (40) in den Produktdaten (16) kompatibel ist.

8. Verfahren nach einem der Ansprüche 1 bis 7,
wobei die Patientendaten (20) das Ergebnis eines Bluttests (36), insbesondere AB0-Bedside-Test, enthalten,
wobei das Endgerät (10) prüft, ob das Ergebnis des Bluttests (36) mit einer Patienten-Blutgruppe (48) in den Patientendaten (20) kompatibel ist.

9. Verfahren nach Anspruch 7 oder 8,
wobei das Ergebnis des Bluttests (36) als eine manuelle Eingabe mittels eines Eingabeelements (34) des Endgeräts (10) erfasst wird.

10. Verfahren nach einem der Ansprüche 1 bis 9,
wobei die Produktdaten (16) eine Produkt-Blutgruppe (40) und einen Produkttyp (42) enthalten,
wobei als eine Patienten-Blutgruppe (48) angebbar ist, dass diese unbekannt ist, in welchem Fall das Endgerät (10) in einen Notfallmodus umschaltet, in welchem das Endgerät (10) die Produktdaten (16) erfasst und prüft, ob in Anbetracht des Produkttyps (42) und der Produkt-Blutgruppe (40) das Blutprodukt (2) allgemeinverträglich ist.

11. Endgerät (10), welches ausgebildet ist, ein Verfahren gemäß einem der Ansprüche 1 bis 10 auszuführen.

12. Endgerät (10) nach Anspruch 11,
wobei dieses ein mobiles Endgerät ist, insbesondere Smartphone, Tablet, PDA oder dergleichen.

13. Computerprogramm, welches Befehle aufweist, welche bewirken, dass ein Endgerät (10) gemäß Anspruch 11 oder 12 die Verfahrensschritte eines Verfahrens gemäß einem der Ansprüche 1 bis 10 ausführt.
